# EUROPEAN PATENT APPLICATION

(11) **EP 3 415 523 A1**
(43) Date of publication of application: **19.12.2018**
(21) Application number: 17176199.2
(22) Date of filing: 15.06.2017
(51) Int. Cl.: C07H 19/06, C07D 311/18, C07D 475/08, A61K 31/352, A61K 31/4375, A61K 31/7068

(54) **COMBINATION MEDICAMENT COMPRISING A PRODRUG AND INHALABLE CATALYST**

(71) Applicant: ETH Zurich, 8092 Zurich (CH)
(72) Inventor: Stark, Wendelin J., 4900 Langenthal (CH); Herzog, Antoine, 8050 Zurich (FR)

(57) **Abstract**

The invention relates to a prodrug compound consisting of a drug moiety comprising an amine or hydroxyl moiety and a protecting group moiety covalently linked to the amine or hydroxyl moiety. The drug moiety is effective in the treatment of cancer when the drug moiety is separated from the protecting group moiety. The protecting group moiety is cleavable from the amine or hydroxyl moiety by reaction with molecular hydrogen (H₂) in the presence of a catalyst. The invention further relates to a combination medicament comprising the prodrug, a catalyst effecting reductive deprotection of the drug moiety, and optionally a reducing agent. In addition, the prodrug compound or the combination medicament are provided for use in a method of treatment or prevention of cancer.
The protecting group has preferably one of the following formulas:

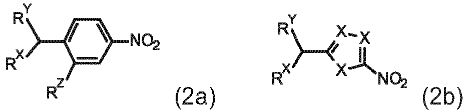

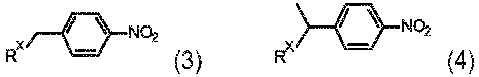

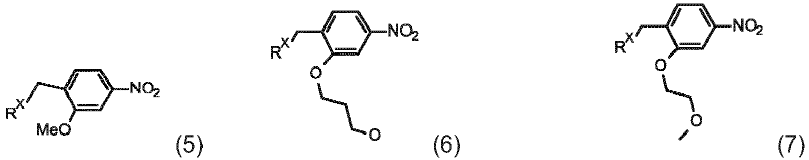

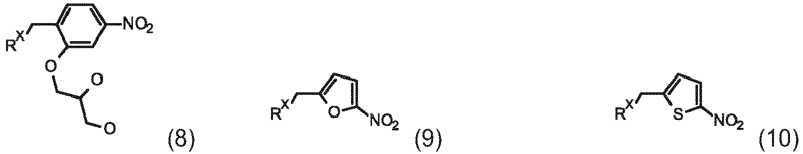

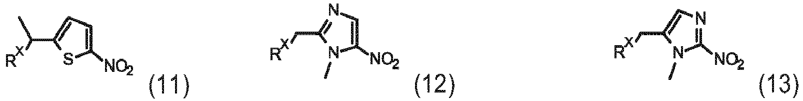

## Description

### Background

A generic problem of anti-cancer treatments is their non-specific mode of action. The molecular mechanisms involved in anti-cancer therapy share a high degree of similarity between healthy and cancer cells, thereby making chemotherapies cytotoxic to cancer cells as well as healthy cells. The destruction of healthy cells (especially rapidly dividing cells) by untargeted chemotherapies causes severe side effects.

These side effects prevent chemotherapies from being suitable for physically weak patients. The life of patients is drastically impacted by a chemotherapy (e.g. by hair loss, pain and fatigue). Physicians have to deal with a trade-off between the treatment efficacy, which increases with higher doses, and the necessity to ensure that the side effects are still bearable. Many patients are not treated to the maximal effective concentration of the drug or the combination of drugs they are being given.

Despite extensive research efforts in the last decades, lung cancer remains a staggering cause of human deaths and accounts for around 25% of cancer deaths, making it the deadliest form of cancer.

To improve treatment efficacy, two main strategies have been followed. First, cancer-specific mechanisms have been identified. New targets were defined (e.g. growth factor, signalling molecules, modulator of apoptosis) and the so-called targeted chemotherapies emerged. The second strategy was to improve the drug delivery by various methods such as antibody drug conjugates or drug delivery through nanoparticles. More specifically, for tumour hypoxia, bioreductive prodrugs such as tirapazamine were synthesised in order to take advantage of the reductive environment of hypoxic pockets (regions where the oxygen content is very low).

Up to now, all those attempts have not been successful and the main treatment for SCLC remains a combination of the untargeted drugs cisplatin (or carboplatin) and etoposide with a low prognosis (median survival rate of 8-10 months for patients with extensive-stage disease which represents two thirds of SCLC patients).

Because most SCLCs patient exhibit a positive initial response to chemotherapies, one strategy to increase the time of response and the survival rate would be to go to higher doses. Unfortunately, the untargeted nature of the drugs currently used make this approach not viable since it would dramatically increase the side effects.

Based on the above-mentioned state of the art, the objective of the present invention is to provide means and methods to enable a high concentration of active chemotherapeutic drugs at the tumour site, while reducing the systemic side effects. This objective is attained by the claims of the present specification.

### Detailed description of the invention

A first aspect of the invention provides a prodrug compound or a pharmaceutically acceptable salt thereof, wherein said prodrug compound consists of
a. a drug moiety comprising an amine or hydroxyl moiety, wherein the drug moiety is effective in the treatment of cancer when the drug moiety is separated from the rest of the prodrug compound,
b. a protecting group moiety covalently linked to the amine or hydroxyl moiety, wherein the protecting group moiety is cleavable from the amine or hydroxyl moiety by reaction with molecular hydrogen (H₂) in the presence of a catalyst.

In certain embodiments, the drug moiety has a molecular mass of more than 160 u but less than 1000 u, particularly less than 700 u, more particularly less than 500 u, comprises up to five hydrogen bond donators (e.g., oxygen and or nitrogen atoms with one H attached), up to ten hydrogen bond acceptors (e.g., oxygen or nitrogen atoms) and is characterized by an octanol-water partition coefficient logP of below 5.6 (any of these characteristics applied to the isolated drug moiety, without regard to the rest of compound). These are the so-called "Lipinski" rules of 5 (originally, referring to molecules between 160 and 500 u) for drug-like compounds.

In certain embodiments, the protecting group moiety is cleavable from the amine or hydroxyl moiety by reaction with molecular hydrogen (H₂) only in the presence of a catalyst, and not in the absence of a catalyst.

In certain embodiments, the protecting group moiety is cleavable from the amine or hydroxyl moiety by reaction with molecular hydrogen (H₂) only in the presence of a catalytic amount of platinum or palladium.

In certain embodiments, the drug moiety is known to interact with a therapeutic target. The drug moiety comprises an interaction site that is the site where the drug moiety interacts with the active site of the therapeutic target. The amine or hydroxyl moiety is located in or near the interaction site of the drug moiety. "near" in this context signifies a relative spatial proximity within the molecule, i.e. within 5Å, 10Å or 15Å distance from an atom demonstrated to participate directly in the interaction between the drug moiety and its target.

In certain embodiments, the drug moiety or its pharmaceutically acceptable salt is effective in the treatment of lung cancer. In certain embodiments, the drug moiety or its pharmaceutically acceptable salt is effective in the treatment of non-small cell lung cancer (NSCLC). In certain embodiments, the drug moiety or its pharmaceutically acceptable salt is effective in the treatment of small cell lung cancer (SCLC).

The drug moiety which is covalently linked to the protecting group moiety cannot fit in the active site of its therapeutic target, thereby ensuring that no cells are killed until deprotection occurs at the target site. Because the deprotection requires a catalyst, e.g. a platinum nanocatalyst, the spatial location of which can be controlled through the administration, a higher local concentration can be obtained.

In the chemireductive prodrug system of the invention, a drug can be administered systemically in high doses without causing the increased side effects usually associated with high doses. The drug is protected when it travels through the patient's circulatory system and is only transformed into its active form where and when needed. The current state of the art consists in giving the patient either the active form itself or a prodrug that is rapidly metabolized to its active counterpart. This results in severe side-effects due to the non-specificity of the drug which can interact with cancer and healthy cells. The chemireductive prodrug system of the invention provides a way to reduce side-effects for the patients compared to current treatments. One advantage is that the life of a patient is less negatively affected by side-effects. Another advantage is that the efficiency of a treatment can be increased without increasing the side-effects.

According to the invention, first an anti-cancer drug having an amine group or a hydroxyl group is chemically protected with a protecting group. Thus protected, the drug cannot interact with either healthy or cancer cells. It can diffuse more easily through lipophilic tissues than its deprotected counterpart. The protected drug can be systemically administered (e.g. ingested) and travel freely through the body without generating significant side-effects.

Two components are required to deprotect the drug: a catalyst and a reducing agent. An example of a suitable reducing agent is molecular hydrogen. The catalyst can be applied locally. One possible form of administration is injection. Another possible form of administration is inhalation of an aerosol. The reducing agent can be administered by inhalation.

This dual requirement is an advantage since the administration of hydrogen as the reducing agent can easily be controlled, thereby making the deprotection tunable. By breathing in more hydrogen, more molecules will be deprotected. The use of a catalyst implies that the deprotection occurs only at controlled location (where the catalyst was applied). In summary, the active form of the drug has high local concentrations, its systemic concentration is low, and the concentration can be controlled in time by hydrogen delivery.

In certain embodiments, the protecting group moiety is selected from the group consisting of
- 4-nitrobenzylcarbamates,
- 1-(4-nitrophenyl)ethylcarbamate,
- 2-methoxy-4-nitrobenzylcarbamate,
- 2-(2-methoxyethoxy)-4-nitrobenzylcarbamate,
- 2-(2,3-dihydroxypropoxy)-4-nitrobenzylcarbamate,
- 2-(3-hydroxypropoxy)-4-nitrobenzylcarbamte,
- (5-nitrothiophen-2-yl)methylcarbamate,
- 1-(5-nitrothiophen-2-yl)ethylcarbamate,
- (1-methyl-2-nitro-1H-imidazol-5-yl)methylcarbamate,
- (1-methyl-5-nitro-1 H-imidazol-2-yl)methylcarbamate,
- (5-nitrofuran-2-yl)methylcarbamate,
- (5-methoxy-1,3-dimethyl-4,7-dioxo-4,7-dihydro-1H-indol-2-yl)methylcarbamate and
- ethyl 4-((carbamoyloxy)methyl)-1-methyl-5-nitro-1H-pyrrole-3-carboxylate.

In certain embodiments, the protecting group moiety is p-nitrobenzyloxycarbonyl. The molecule resulting from the deprotection of a drug moiety protected with p-nitrobenzyloxycarbonyl, namely p-toluidine, has been investigated by the European Commission (2013, Recommendation from the Scientific Committee on Occupational Exposure Limits for 4-Aminotoluene (p-Toluidine)). The reported LD₅₀ values (several hundreds of mg/kg for rodents) are much more than the amounts generated by the method of the present invention. The molecule was also shown to be neither mutagenic nor genotoxic in an vitro test on mammalian cells.

In certain embodiments, the catalyst is a platinum group metal selected from ruthenium, rhodium, palladium, osmium, iridium and platinum in oxidation state 0.

In certain embodiments, the catalyst is platinum.

In certain embodiments, the catalyst is palladium.

In certain embodiments, the drug moiety is selected from the list comprising afatinib dimaleate, alectinib, brigatinib, ceritinib, crizotinib, docetaxel, doxorubicin hydrochloride, erlotinib hydrochloride, etoposide, etoposide phosphate, everolimus, gefitinib, gemcitabine hydrochloride, methotrexat, osimertinib, paclitaxel, pemetrexel disodium, topotecan hydrochloride, irinotecan and vinorelbine tartrate.

In certain embodiments, the prodrug compound is characterized by a formula (1) 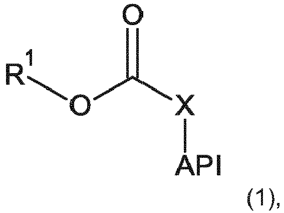 wherein API represents the drug moiety, X represents O or N (wherein API comprises a secondary amine represented by X and two bonds connect X with the API scaffold) or NH or NR² with R² being selected from C₁ to C₆ alkyl, and R¹-OCO represents the protecting group moiety.

In certain embodiments, X is a secondary amine comprised within the drug moiety, and is liberated to NH upon hydrogenation, such as, for example, the NH bridging two aromatic rings in erlotinib and gefitinib.

In certain embodiments, X is a primary amine comprised within the drug moiety, and is liberated to NH₂ upon hydrogenation, such as, for example, the NH₂ in doxorubicin or gemcitabine.

In certain embodiments, R¹ is selected from a moiety characterized by any one of formulae (2a), (2b), (3), (4), (5), (6), (7), (8), (9), (10), (11), (12), (13), (14) or (15) 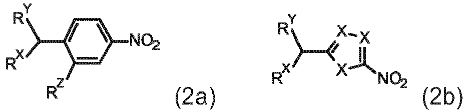 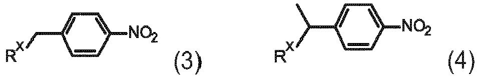 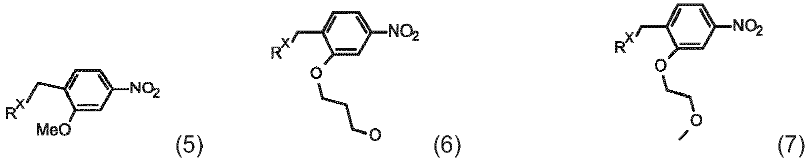 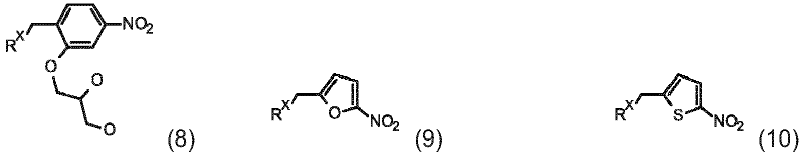 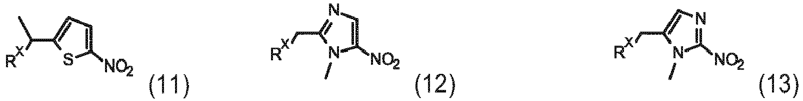 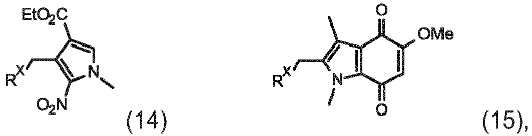 wherein
R^{Y} is a substituted or unsubstituted alkyl, particularly a substituted or unsubstituted C₁ to C₁₂ alkyl, more particularly a substituted or unsubstituted C₁ to C₆ alkyl,
R^{Z} is selected from C₁ to C₆ alkyl
X is selected from O, S, NH or NR³ with R³ being selected from C₁ to C₆ alkyl and
R^{X} represents OCO-X-API.

In certain embodiments, API is selected from the list comprising a compound characterized by any one of formulae (16), (17), (18), (19), (20), (21), (22), (23), (24), (25), (26), (27), (28), (29), (30), (31), (32) or (33).

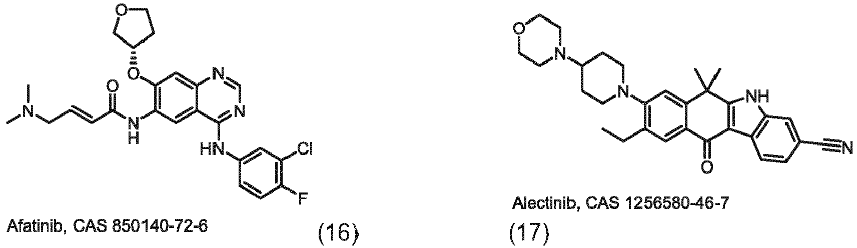

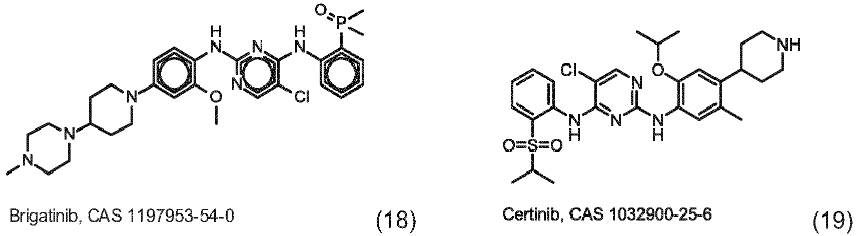

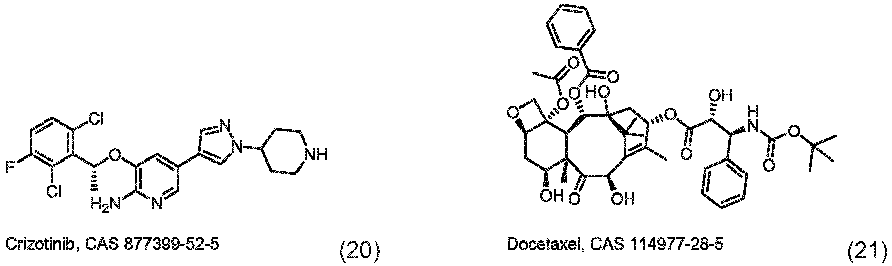

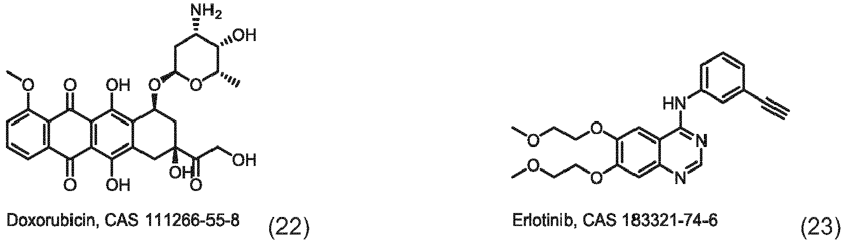

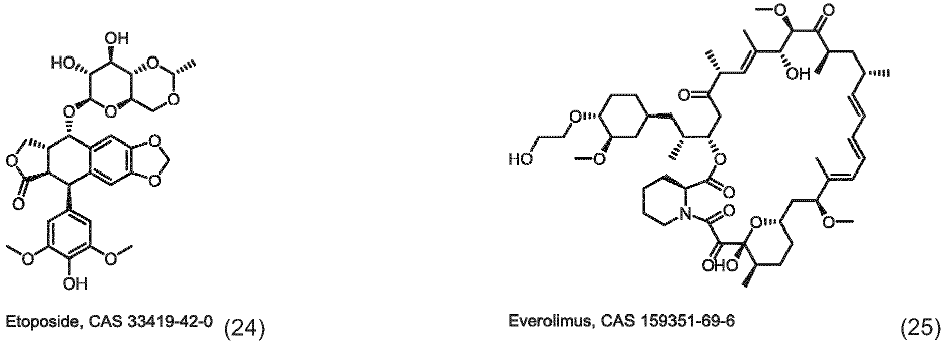

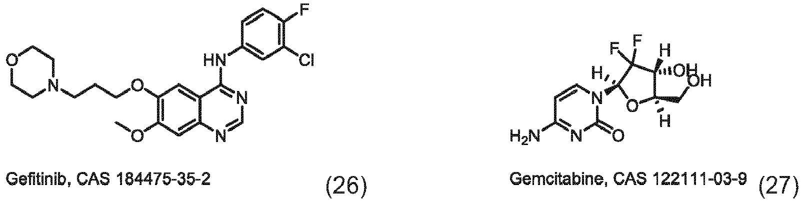

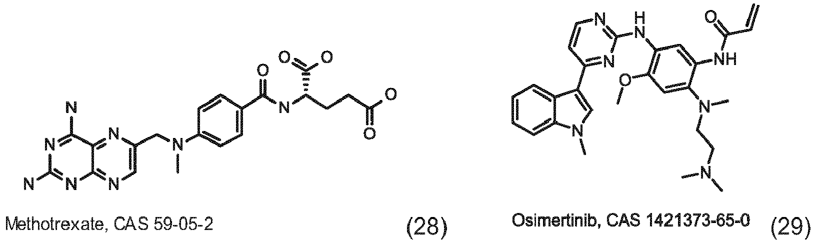

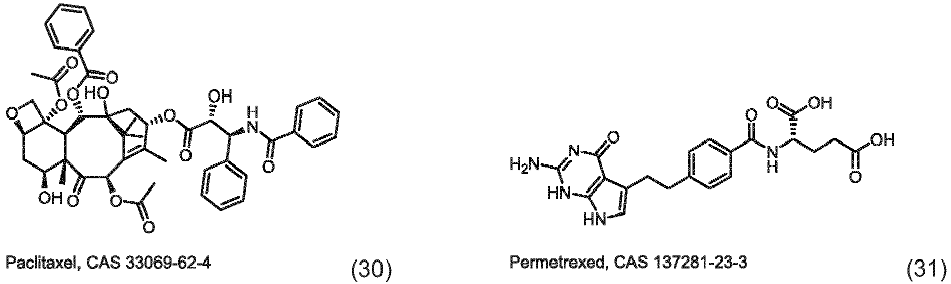

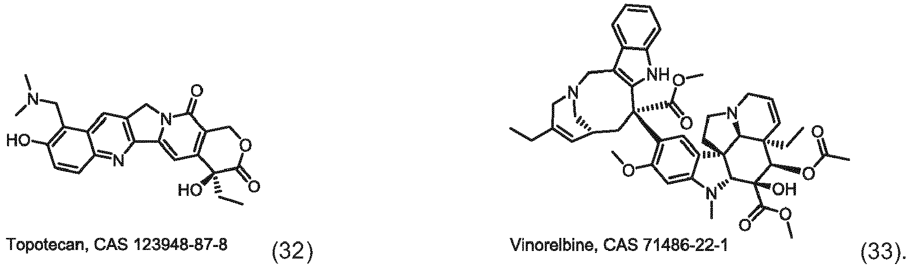

In certain embodiments, the prodrug compound is characterized by any one of formulae (16a), (17a), (18a), (18b) (19a), (20a), (21a), (22a), (23a), (24a), (24b), (24c), (25a), (26a), (27a), (28a), (28b), (29a), (30a), (31 a), (32a), (32b) or (33a).

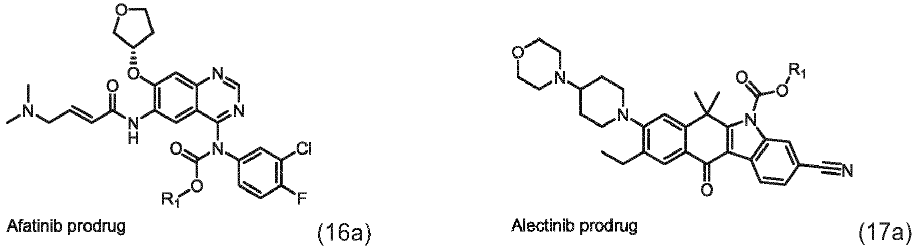

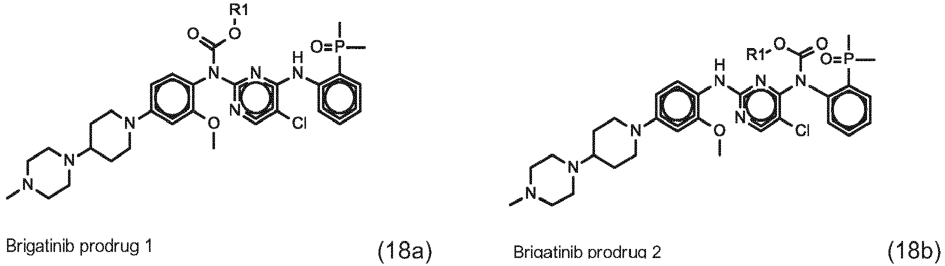

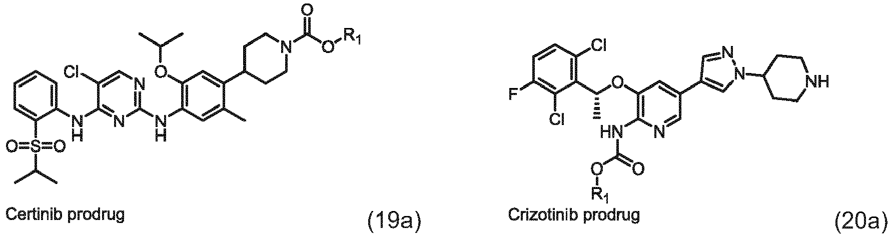

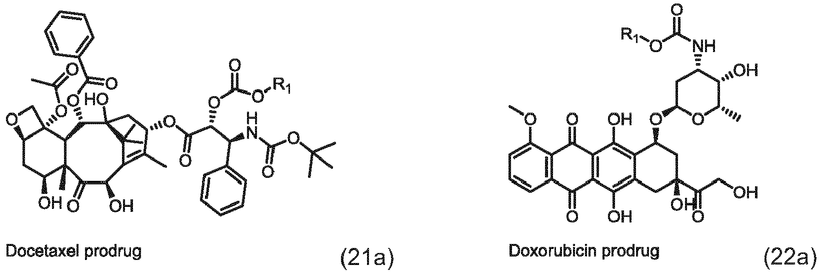

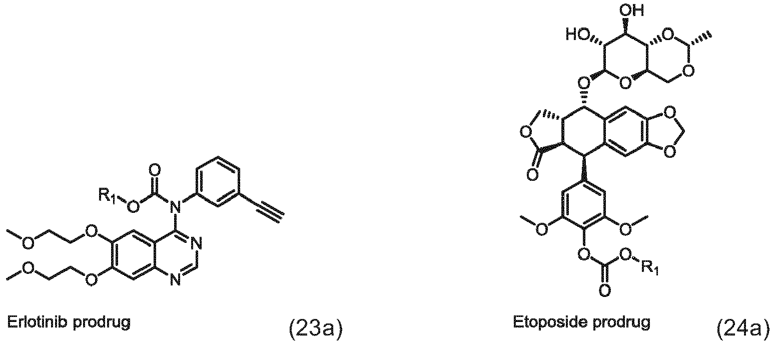

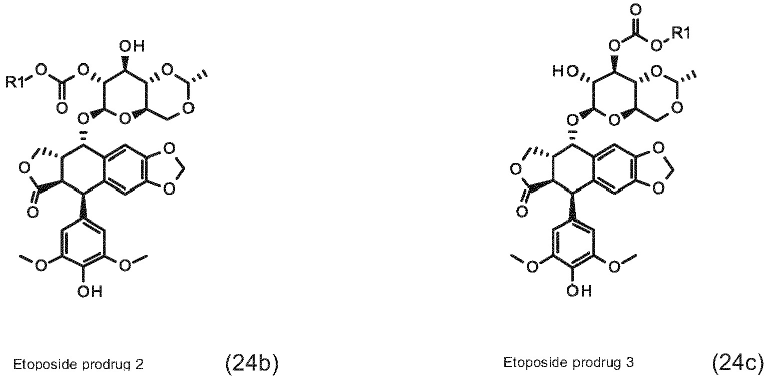

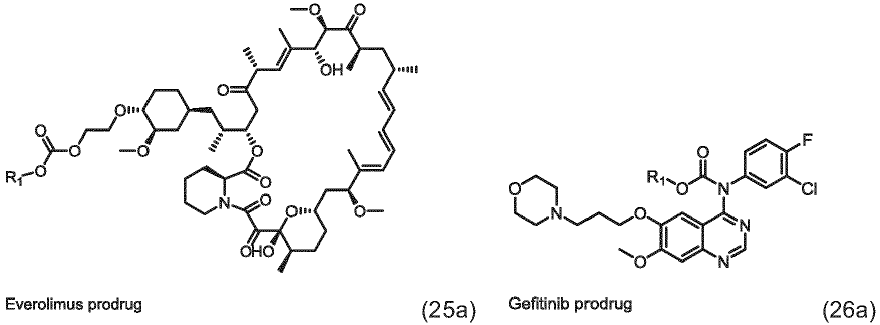

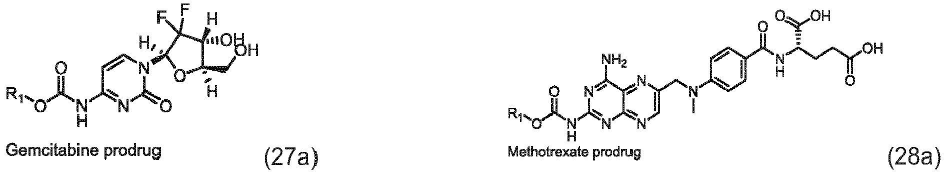

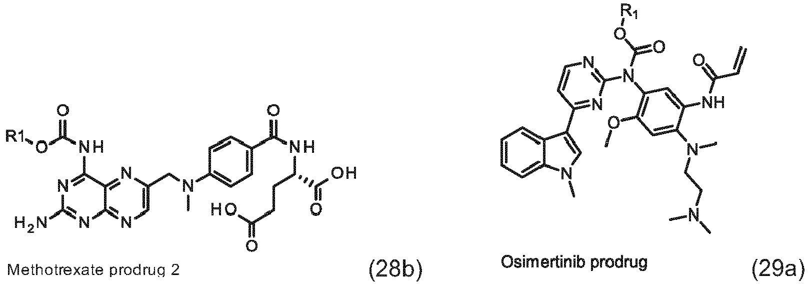

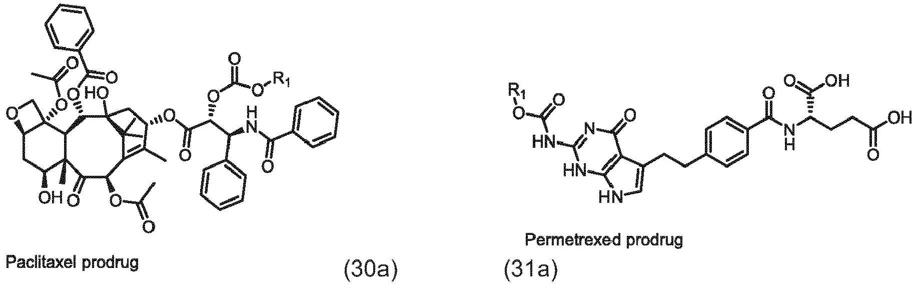

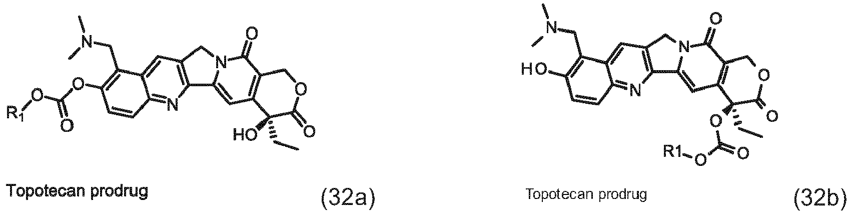

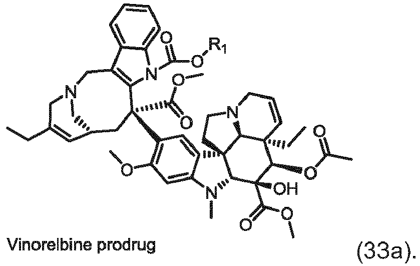

A second aspect of the invention provides a combination medicament comprising
a. the prodrug or its pharmaceutically acceptable salt according to the first aspect of the invention,
b. a catalyst effecting reductive deprotection of the drug moiety in the presence of H₂, and optionally
c. a reducing agent.

In certain embodiments of this aspect of the invention, the reducing agent is a gas mixture, in particular a gas mixture comprising hydrogen or deuterium.

In certain embodiments of this aspect of the invention, the reducing agent is hydrogen H₂.

In certain embodiments of this aspect of the invention, the reducing agent is a gas mixture comprising up to 100% hydrogen.

In certain embodiments of this aspect of the invention, the gas mixture comprises ≤ 4% oxygen and 0% - 96% hydrogen.

In certain embodiments of this aspect of the invention, the gas mixture comprises ≥ 4% oxygen and 0% - 5% hydrogen.

Hydrogen is not toxic to humans and is already used by deep-diver to a concentration up to 96% hydrogen and 4% oxygen (Fife, W. P. 1979, National Oceanic and Atmospheric Administration's Office of Sea Grants and Texas A&M University, Texas).

In certain embodiments of any aspect of the invention, the prodrug compound or its pharmaceutically acceptable salt is formulated for systemic delivery, and the catalyst is formulated for locally restricted delivery.

In certain embodiments of any aspect of the invention, the catalyst is formulated for inhalation.

In certain embodiments of any aspect of the invention, the prodrug compound or its pharmaceutically acceptable salt, as well as the catalyst are formulated for inhalation.

In certain embodiments of any aspect of the invention, the catalyst is a platinum group metal selected from ruthenium, rhodium, palladium, osmium, iridium and platinum in oxidation state 0.

In certain embodiments of any aspect of the invention, the catalyst is selected from platinum and palladium.

In certain embodiments of any aspect of the invention, the catalyst is a nanoparticle comprising, or consisting of, a platinum group metal.

In certain embodiments of any aspect of the invention, the catalyst is a nanoparticle comprising a platinum group metal on a carrier. By way of non-limiting example, the carrier can be a carbon nanoparticle.

In certain embodiments of any aspect of the invention, the catalyst is a nanoparticle with a diameter of between 1 nm and 250 nm, in particular between 2 nm and 20 nm.

In certain embodiments of any aspect of the invention, the catalyst is a nanoparticle comprising catalytic platinum.

In certain embodiments of any aspect of the invention, the catalyst is a nanoparticle consisting of catalytic platinum.

The catalyst may be provided in the form of a nanoparticle in suspension. The catalyst may be formulated as an aerosol.

In certain embodiments of any aspect of the invention, the catalyst is a platinum nanoparticle with a diameter between 1 nm and 250 nm.

In certain embodiments of any aspect of the invention, the catalyst is a platinum nanoparticle with a diameter between 2 nm and 20 nm.

In certain embodiments of any aspect of the invention, the catalyst is a platinum nanoparticle with a diameter of approximately 3 nm.

Another aspect of the invention provides the prodrug compound or its pharmaceutically acceptable salt according to the first aspect of the invention, or the combination medicament according to the second aspect of the invention for use in a method of treatment or prevention of cancer.

In certain embodiments of this aspect of the invention, the method of treatment or prevention comprises the steps of
a. inhalation of a catalyst effecting the reductive deprotection of the drug moiety;
b. administration of the prodrug compound or its pharmaceutically acceptable salt; and optionally
c. inhalation of a reducing agent, in particular hydrogen.

In certain embodiments of this aspect of the invention, the reducing agent is a gas mixture, in particular a gas mixture comprising hydrogen or deuterium.

In certain embodiments of this aspect of the invention, the reducing agent is hydrogen H₂.

In certain embodiments of this aspect of the invention, the prodrug compound or its pharmaceutically acceptable salt is administered systemically.

In certain embodiments of this aspect of the invention, the prodrug compound or its pharmaceutically acceptable salt is administered by inhalation.

In practice, the patient inhales an aerosol formulation of the catalyst, e.g. of platinum nanoparticles. The nanoparticles reside in the airways thereby conferring a spatial control. In a second step, the prodrug compound is administered. Finally, a hydrogen-containing gas mixture is inhaled to trigger the chemical deprotection of the prodrug. The control of the hydrogen administration provides a temporal control and can thus fine-tune the dose profile.

In certain embodiments of this aspect of the invention, the cancer is lung cancer.

In certain embodiments of this aspect of the invention, the lung cancer is non-small cell lung cancer (NSCLC) or small cell lung cancer (SCLC).

In certain embodiments of this aspect of the invention, the lung cancer is small cell lung cancer (SCLC).

SCLC accounts for 10-15% of lung cancers and is an extremely aggressive form of cancer with only rare remission and a median survival of 2-4 months for patients without treatment. SCLCs often reside in hypoxic pockets (regions where the oxygen content is very low). More than 60% of patients develop severe local hypoxia which has several consequences with respect to the treatment efficiency. First, radiotherapy, which works by producing free radicals, turns out to be inefficient due to the rapid reduction of the free radicals. Moreover, hypoxia results in the so-called Warburg effect, which consists in the switch from aerobic respiration to glycolysis as well as an upregulation of many genes (e.g. the hypoxia-inducible factors gene). Consequently, cells located in hypoxic regions have an increased resistance to chemotherapy and an enhanced proliferation.

The method according to the invention enables targeting tumours in hypoxic pockets, because the catalytic reaction is selective to reducing environments such as in the hypoxic pockets.

Another complication of tumours in hypoxic pockets results from the fact that the tumour cells are further apart from blood vessels. This increases the diffusion length required to reach the interior of hypoxic regions, implying that only a reduced fraction of the molecules can reach their target. the protected prodrug of the invention can diffuse more easily through lipophilic tissues than its deprotected counterpart. Therefore, a higher fraction of molecules can reach the tumour.

The system of a protected drug generating less side-effects than its unprotected counterpart and requiring a catalyst for deprotection can be applied to other cancers and other diseases as well.

According to yet another aspect of the invention, a dosage form comprising the prodrug compound or its pharmaceutically acceptable salt as specified by the first aspect of the invention is provided, particularly for use in a method of treatment or prevention of cancer. Optionally, a pharmaceutically acceptable carrier and/or excipient may be present.

According to an alternative aspect of the invention, the prodrug compound or its pharmaceutically acceptable salt as specified in the first aspect of the invention is provided as a medicament, particularly a medicament formulated for use in the treatment or prevention of cancer. The medicament comprises the prodrug compound or its pharmaceutically acceptable salt as specified in the first aspect of the invention alone or together with one or more pharmaceutically acceptable excipients or carriers.

In certain embodiments, the dosage form or the medicament are formulated for systemic administration. In certain embodiments, the dosage form or the medicament are formulated for ingestion. In certain embodiments, the dosage form or the medicament are formulated for parenteral administration, such as intravenous, intraperitoneal, intramuscular, intra-arterial or subcutaneous administration. In certain embodiments, the dosage form or the medicament are formulated for inhalation.

### Terms and definitions

The term catalyst in the context of the present specification relates to a substance that causes an increase in the rate of a chemical reaction, but is not itself involved in the reaction. The catalyst is not consumed in the catalyzed reaction and can continue to act repeatedly. In the absence of the catalyst, the chemical reaction does not occur, or only at a very low rate.

The term nanocatalyst in the context of the present specification relates to a catalyst provided in the form of a nanoparticle.

The term systemic administration in the context of the present specification relates to a route of administration of a compound into the circulatory system of a subject.

The term protecting group in the context of the present specification is synonymous with the term protective group and relates to a covalent modification of a functional group of a molecule. The protecting group is introduced into the thus protected molecule by a chemical reaction. The term functional group in the context of the present specification relates to a group of atoms within a molecule that is responsible for the characteristic chemical reactions of this molecule. In the context of the present specification these functional groups are amine moieties or hydroxyl moieties.

The term deprotection in the context of the present specification relates to a chemical reaction removing a protecting group from a protected molecule (prodrug), thereby releasing an unprotected molecule (drug moiety, active pharmaceutical ingredient). In the context of the present invention, a catalyst is necessary for the deprotection reaction to occur.

The term platinum group metal in the context of the present specification relates to the metals ruthenium, rhodium, palladium, osmium, iridium and platinum in oxidation state 0.

The term hydrogen in the context of the present specification relates to molecular hydrogen (H₂).

The term deuterium in the context of the present specification relates to molecular deuterium (¹H₂ or D₂).

In the context of the present specification, the abbreviation API stands for active pharmaceutical ingredient.

In the context of the present specification, the term lung cancer relates to a condition characterized by uncontrolled cell growth in the tissues of the lung. The term encompasses both primary lung cancers, which originate in the lung, and secondary lung cancers. The two main types of primary lung cancers are small cell lung cancer (SCLC) and non-small cell lung cancer (NSCLC). Secondary lung cancers develop from metastatic cells of tumours from other parts of the body (e.g. breast cancer) that have spread to the lung.

The skilled person is aware that any specifically mentioned drug may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

The term *C₁-C₄ alkyl* in the context of the present invention signifies a saturated linear or branched hydrocarbon having 1, 2, 3 or 4 carbon atoms, wherein in certain embodiments one carbon-carbon bond may be unsaturated and one CH₂ moiety may be exchanged for oxygen (ether bridge) or nitrogen (NH, or NR with R being methyl, ethyl, or propyl; amino bridge). Non-limiting examples for a C₁-C₄ alkyl are methyl, ethyl, propyl, prop-2-enyl, n-butyl, 2-methylpropyl, *tert*-butyl, but-3-enyl, prop-2-inyl and but-3-inyl. In certain embodiments, a C₁-C₄ alkyl is a methyl, ethyl, propyl or butyl moiety.

A C₁-C₆ alkyl in the context of the present invention signifies a saturated linear or branched hydrocarbon having 1, 2, 3, 4, 5 or 6 carbon atoms, wherein one carbon-carbon bond may be unsaturated and one CH₂ moiety may be exchanged for oxygen (ether bridge) or nitrogen (NH, or NR with R being methyl, ethyl, or propyl; amino bridge). Non-limiting examples for a C₁-C₆ alkyl include the examples given for C₁-C₄ alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, pent-4-inyl, 3-methyl-2-pentyl, and 4-methyl-2-pentyl. In certain embodiments, a C₅ alkyl is a pentyl moiety and a C₆ alkyl is a hexyl or cyclohexyl moiety.

The term *unsubstituted Cₙ alkyl* when used herein in the narrowest sense relates to the moiety -CₙH₂ₙ- if used as a bridge between moieties of the molecule, or -CₙH₂ₙ₊₁ if used in the context of a terminal moiety.

The term substituted alkyl refers to an alkyl according to the above definition that is modified by one or several amine or hydroxyl groups NH₂, NHR, NR² or OH, wherein the R substituent as used in the current paragraph, different from other uses assigned to R in the body of the specification, is methyl, ethyl or propyl unless otherwise specified. An alkyl having more than one carbon may comprise more than one amine or hydroxyl. Unless otherwise specified, the term "substituted alkyl" refers to alkyl in which each C is only connected to at most one amine or hydroxyl group, in addition to bonds to the alkyl chain, terminal methyl, or hydrogen.

Where used in the context of chemical formulae, the following abbreviations may be used: Me is methyl CH₃, *Et* is ethyl -CH₂CH₃, *Prop* is propyl -(CH₂)₂CH₃ (n-propyl, n-pr) or -CH(CH₃)₂ (iso-propyl, i-pr), *but* is butyl -C₄H₉, -(CH₂)₃CH₃, -CHCH₃CH₂CH₃, -CH₂CH(CH₃)₂ or -CH(CH₃)₃.

A comprehensive review of modern protecting group chemistry, particularly as it pertains to the compounds disclosed herein, is available in Peter G. M. Wuts, Greene's Protective Groups in Organic Synthesis, 5th Edition, Wiley 2014.

US 6693178 B2 - "Protecting groups useful in the synthesis of polysaccharides, natural products, and combinatorial libraries" and US 20160024143 A1 - "Deprotection method" are incorporated herein by reference.

Wherever alternatives for single separable features such as, for example, drug moiety, a protecting group or a catalyst are laid out herein as "embodiments", it is to be understood that such alternatives may be combined freely to form discrete embodiments of the invention disclosed herein. Thus, any of the alternative embodiments of a drug moiety may be combined with any of the alternative embodiments of a protecting group, any of the alternative embodiments of a prodrug may be combined with any of the alternative embodiments of a catalyst, and these combinations may be combined with any medical indication or method of treatment mentioned herein.

The invention is further illustrated by the following examples, items and figures, from which further embodiments and advantages can be drawn. These examples, items and figures are meant to illustrate the invention but not to limit its scope.

### Brief description of the figures

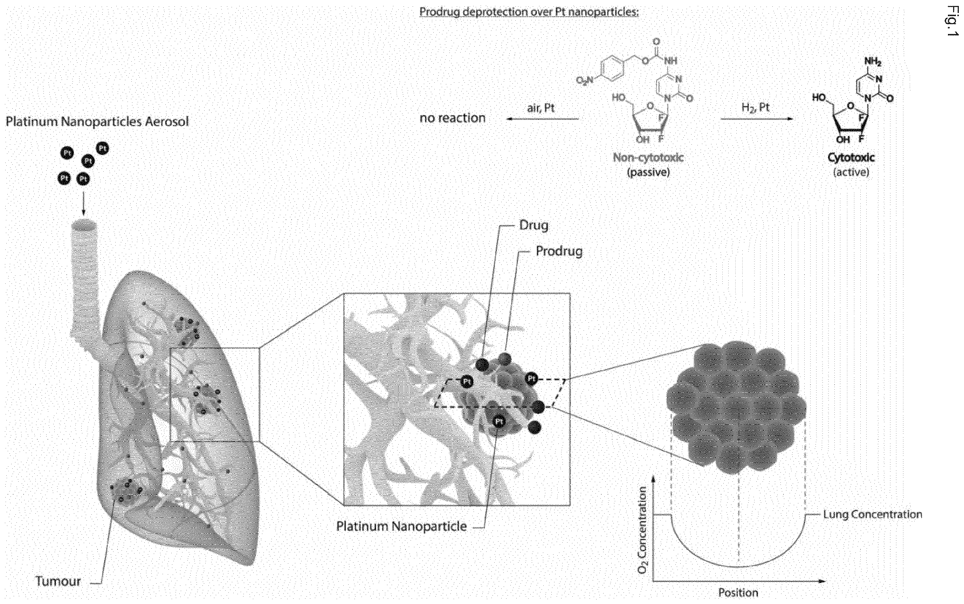

Fig. 1 shows an overview of the treatment principles of the invention. A passive prodrug is administered and is deprotected in the lungs over platinum nanoparticles if in presence of hydrogen. The catalytic reaction is selective to reducing environments such as in the hypoxic pockets of tumours. The concentration of the active form of the drug is higher in the direct vicinity of the tumour (spatial control) and can be controlled by the hydrogen supply (temporal control). The concentration gradient around tumours lead to a lowering of the side effects.

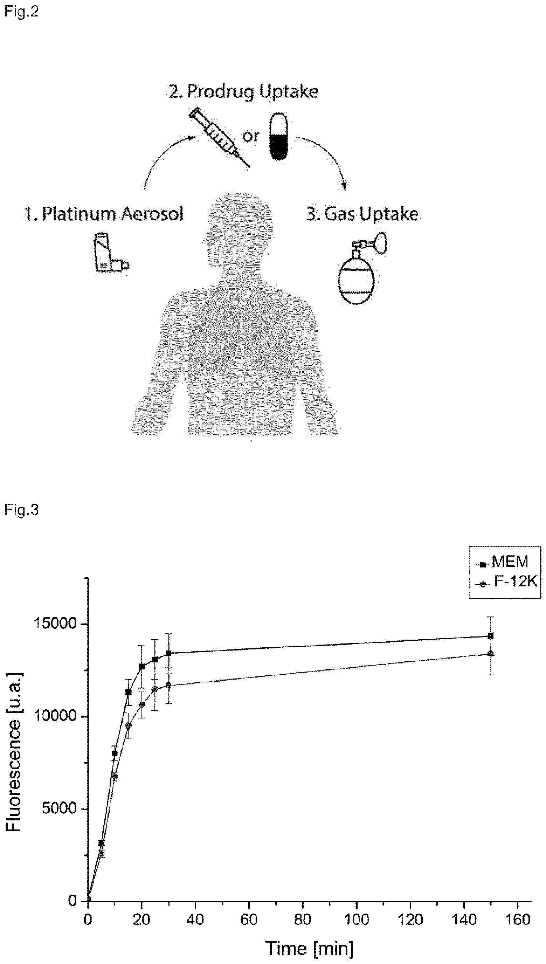

Fig. 2 shows a schematic of the treatment process. The patient initially inhales an aerosol of platinum nanoparticles. This step is followed by the prodrug uptake in any of common route of administration. Finally, an air mixture containing hydrogen is breathed thereby triggering the deprotection of the prodrug.
Fig. 3 shows the kinetics of the deprotection reaction of the protected coumarin followed by fluorescence measurements (excitation wavelength: 540 nm, emission wavelength: 590 nm).
The reaction half-life is about 10 min.
Fig. 4 shows the "interval kinetics" of the deprotection reaction of the protected coumarin followed by fluorescence measurements (excitation wavelength: 540 nm, emission wavelength: 590 nm). The striped bands indicate the intervals where cells were experiencing the hydrogen atmosphere.
Fig. 5 shows the kinetics of the deprotection reaction of the prodrug followed by HPLC-MS measurements. The reaction half-life is about 10 min.
Fig. 6 shows a dose response study of cell viability after 3 days of treatment on A549 cells.
Fig. 7 shows a viability assay after 3 days of treatment of A549 cells with protected gemcitabine. The experiment was done with 0.05 µM of drug/prodrug concentration and 5 ppm of a platinum nanoparticles solution
Fig. 8 shows a viability assay after 3 days of treatment of A549 cells with protected gemcitabine. The experiment was done with 0.05 µM of drug/prodrug concentration and 10 ppm of a platinum nanoparticles solution.

### Items

1. A prodrug compound consisting of
   a. a drug moiety comprising an amine or hydroxyl moiety,
   b. a protecting group moiety covalently linked to said amine or hydroxyl moiety,
   wherein the protecting group moiety is cleavable from said amine or hydroxyl moiety by reaction with molecular hydrogen in the presence of a catalyst,
   wherein said drug moiety is effective in the treatment of cancer when the drug moiety is separated from the protecting group moiety,
   or a pharmaceutically acceptable salt of said prodrug compound.
2. The prodrug compound or its pharmaceutically acceptable salt of item 1, wherein said drug moiety is known to interact with a therapeutic target, and said drug moiety comprises an interaction site that is the site where said drug moiety interacts with said therapeutic target, and said amine or hydroxyl moiety is located in or near said interaction site.
3. The prodrug compound or its pharmaceutically acceptable salt of item 1 or 2, wherein said protecting group moiety is selected from the group consisting of 4-nitrobenzylcarbamate, 1-(4-nitrophenyl)ethylcarbamate, 2-methoxy-4-nitrobenzylcarbamate, 2-(2-methoxyethoxy)-4-nitrobenzylcarbamate, 2-(2,3-dihydroxypropoxy)-4-nitrobenzylcarbamate, 2-(3-hydroxypropoxy)-4-nitrobenzylcarbamte, (5-nitrothiophen-2-yl)methylcarbamate, 1-(5-nitrothiophen-2-yl)ethylcarbamate, (1-methyl-2-nitro-1H-imidazol-5-yl)methylcarbamate, (1-methyl-5-nitro-1H-imidazol-2-yl)methylcarbamate, (5-nitrofuran-2-yl)methylcarbamate, (5-methoxy-1,3-dimethyl-4,7-dioxo-4,7-dihydro-1H-indol-2-yl)methylcarbamate and ethyl 4-((carbamoyloxy)methyl)-1-methyl-5-nitro-1 H-pyrrole-3-carboxylate.
4. The prodrug compound or its pharmaceutically acceptable salt according to any one of the preceding items, wherein said catalyst is a platinum group metal selected from ruthenium, rhodium, palladium, osmium, iridium and platinum.
5. The prodrug compound or its pharmaceutically acceptable salt according to any one of the preceding items, wherein the catalyst is selected from platinum and palladium.
6. The prodrug compound or its pharmaceutically acceptable salt according to any one of the preceding items, wherein said drug moiety is selected from the list comprising afatinib dimaleate, alectinib, brigatinib, ceritinib, crizotinib, docetaxel, doxorubicin hydrochloride, erlotinib hydrochloride, etoposide, etoposide phosphate, everolimus, gefitinib, gemcitabine hydrochloride, methotrexat, osimertinib, paclitaxel, pemetrexel disodium, topotecan hydrochloride, irinotecan and vinorelbine tartrate.
7. The prodrug compound or its pharmaceutically acceptable salt of item 1 to 6, wherein the prodrug compound is characterized by a formula (1) 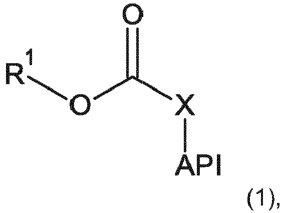 wherein API represents the drug moiety, X represents O or N (wherein API comprises a secondary amine represented by X and two bonds connect X with the API scaffold) or NH or NR² with R² being selected from C₁ to C₆ alkyl, and R¹-OCO represents the protecting group moiety.
8. The prodrug compound or its pharmaceutically acceptable salt according to item 6, wherein R¹ is selected from a moiety characterized by any one of formulae (2a), (2b), (3), (4), (5), (6), (7), (8), (9), (10), (11), (12), (13), (14) or (15) 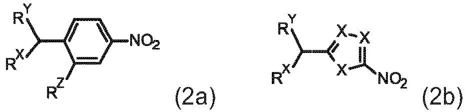 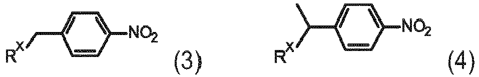 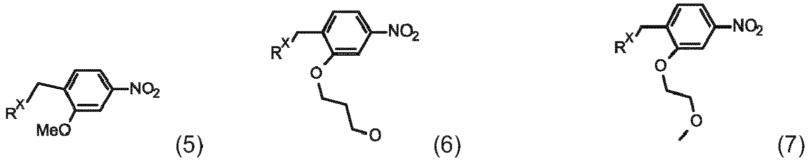 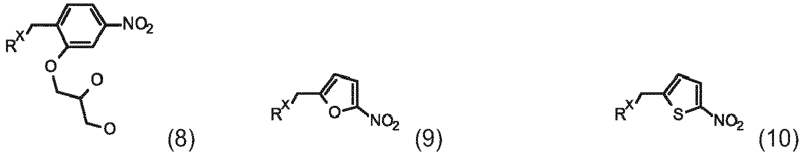 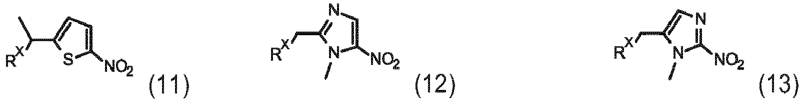 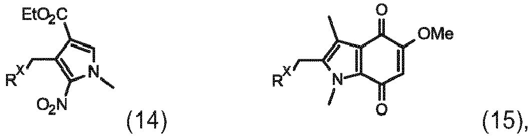 wherein
   R^{Y} is a substituted or unsubstituted alkyl, particularly a substituted or unsubstituted C₁ to C₁₂ alkyl, more particularly a substituted or unsubstituted C₁ to C₆ alkyl,
   R^{Z} is selected from C₁ to C₆ alkyl,
   X is selected from O, S, NH or NR³ with R³ being selected from C₁ to C₆ alkyl and
   R^{X} represents OCO-X-API.
9. The prodrug compound or its pharmaceutically acceptable salt according to item 6 or 8, wherein API is selected from the list comprising a compound characterized by any one of formulae (16), (17), (18), (19), (20), (21), (22), (23), (24), (25), (26), (27), (28), (29), (30), (31), (32) or (33).
10. A prodrug compound characterized by any one of formulae (16a), (17a), (18a), (18b) (19a), (20a), (21 a), (22a), (23a), (24a), (24b), (24c), (25a), (26a), (27a), (28a), (28b), (29a), (30a), (31 a), (32a), (32b) or (33a), or its pharmaceutically acceptable salt.
11. A combination medicament comprising
   a. the prodrug compound or its pharmaceutically acceptable salt according to any one of items 1 to 10,
   b. a catalyst capable of effecting said reductive deprotection of said drug moiety, and optionally
   c. a reducing agent, particularly hydrogen H₂.
12. The combination medicament according to item 11, wherein
   a. said prodrug compound or its pharmaceutically acceptable salt is formulated for systemic delivery, and
   b. said catalyst is formulated for locally restricted delivery, in particular for inhalation.
13. The combination medicament according to item 11, wherein said prodrug compound or its pharmaceutically acceptable salt, and said catalyst are formulated for inhalation.
14. The combination medicament according to any one of items 11 to 13, wherein said reducing agent is a gas mixture, in particular gas mixture containing hydrogen or deuterium.
15. The combination medicament according to any one of items 11 to 14, wherein said catalyst is a platinum group metal selected from ruthenium, rhodium, palladium, osmium, iridium and platinum, in particular from platinum and palladium.
16. The combination medicament according to any one of items 11 to 15, wherein said catalyst is a nanoparticle comprising, or consisting of, catalytic platinum.
17. The prodrug according to any one of items 1 to 10, or a combination medicament according to any one of items 11 to 16 for use in a method of treatment or prevention of cancer.
18. The prodrug or combination medicament for use in a method of treatment or prevention of cancer according to item 17, wherein said method of treatment or prevention comprises the steps of
   a. inhalation of a catalyst effecting said reductive deprotection of said API;
   b. administration of said prodrug; and optionally
   c. inhalation of a reducing agent, in particular inhalation of a gas mixture comprising hydrogen.
19. The prodrug or combination medicament for use in a method of treatment or prevention of cancer according to any one of items 17 to 18, wherein said prodrug is administered systemically.
20. The prodrug or combination medicament for use in a method of treatment or prevention of cancer according to any one of items 17 to 18, wherein said prodrug is administered by inhalation.
21. The prodrug or combination medicament for use in a method of treatment or prevention of cancer according to any one of items 17 to 20, wherein said cancer is selected from primary lung cancer and secondary lung cancer, particularly small cell lung cancer.

### Examples

### Example 1: General experimental procedures

### Kinetics of Deprotection (coumarin)

In a 96-well plate, wells were filled with 90 µL of fresh MEM or F-12K medium containing protected coumarin (110 µM) and DMSO (2% v/v). To treated wells, 10 µL of a platinum nanoparticles solution (1000 ppm, 3 nm diameter) was added and 10 µL of water was added to control wells. The plate was placed in a custom-designed gas chamber. The chamber was placed in the incubator, connected to a gas bottle outside the incubator and to an outlet. The gas chamber was flushed for 0.5 min with hydrogen gas at 1 bar and closed. The plate was incubated 0, 5, 10, 15, 20, 25, 30, 100 min in the gas chamber. After each time point, the plate was taken out of the gas chamber and fluorescence emission was measured using a microplate reader (Tecan; ex: 540 nm; em: 590 nm).

### Interval Kinetics of Deprotection (coumarin)

In a 96-well plate, wells were filled with 100 µL of fresh MEM medium containing protected coumarin (100 µM) and DMSO (2% v/v). To treated wells, 10 µL of a platinum nanoparticles solution (250 ppm or 500 ppm, 3 nm diameter) was added and 10 µL of water was added to control wells. The plate was placed in a custom-designed gas chamber. The chamber was placed in the incubator, connected to a gas bottle outside the incubator and to an outlet. The gas chamber was flushed for 0.5 min with hydrogen gas at 1 bar and closed. The plate was incubated 0.5 min in the gas chamber. The plate was taken out of the gas chamber and left open for 2 min in order to equilibrate with the ambient atmosphere of the room. Fluorescence emission was measured using a microplate reader (Tecan; ex: 540 nm; em: 590 nm).

The plate was placed back in the incubator for 2 min before measuring fluorescence again. Four such cycles were made, followed by two cycles with 1 min incubation in the gas chamber and 2 min of incubation directly in the incubator. Two last cycles were done with 4 min incubation with hydrogen and 2 and 4 min respectively of incubation directly in the incubator.

### Kinetics of Deprotection (gemcitabine)

In a 96-well plate, wells were filled with 100 µL of fresh F-12K medium containing protected gemcitabine (0.1 µM) and DMSO (2% v/v). To treated wells, 10 µL of a platinum concentration (200 ppm, 3 nm diameter) was added and 10 µL of water was added to control wells. The plate was placed in a custom-designed gas chamber. The chamber was placed in the incubator, connected to a gas bottle outside the incubator and to an outlet. The gas chamber was flushed for 1 min with hydrogen gas at 1 bar and closed. The plate was incubated 0, 15, 30, 120 min in the gas chamber before placing them back in the incubator with the control plates. After each iteration, the plate was taken out of the gas chamber and the content of 6 wells were pooled together in an Eppendorf tube. The plate was placed back in the gas chamber and back in the incubator, flushed for 1 min with hydrogen gas at 1 bar and closed until the next sampling. The Eppendorf tubes collected were centrifuge at 21130 g for 1 h and the supernatant was transferred to MS sample vials before measurement with an HPLC-MS (Agilent).

### EC₅₀ Experiments

A549 cells were seeded in 96-well plates at a cell concentration of 2,000 cells/well and incubated overnight before treatment. Each well was then replaced with 100 µL of fresh F-12K medium containing protected gemcitabine (concentration ranging from 10⁴ µM to 10⁻⁴ µM) and DMSO (2% v/v), control wells were replaced with 100 µL of fresh F-12K medium and DMSO (2% v/v). Control and treated plates were incubated for 72 h before PrestoBlue cell viability reagent (10% v/v) was added to each well and the plate incubated for 2 h. Fluorescence emission was measured using a microplate reader (Tecan; ex: 540 nm; em: 590 nm).

### Extracellular Prodrug Activation

A549 cells were seeded in 96-well plates at a cell concentration of 4,000 cells/well and incubated overnight before treatment. Each well was then replaced with 100 µL of fresh F-12K medium containing protected gemcitabine (0.05 µM) and DMSO (2% v/v), control wells were replaced with 100 µL of fresh F-12K medium and DMSO (2% v/v). Plates were incubated for 1 h before 10 µL of a platinum nanoparticles solution (50 ppm, 3 nm diameter) was added to each well and 10 µL of water was added to control wells. Plates were incubated for 1 h before placing them in a custom-designed gas chamber. Control plates were left untouched in the incubator. The chamber was placed in the incubator, connected to a gas bottle outside the incubator and to an outlet. The gas chamber was flushed for 1 min with hydrogen gas at 1 bar and closed. Plates were incubated 2 h in the gas chamber before placing them back in the incubator with the control plates. Control and treated plates were incubated for 72 h before PrestoBlue cell viability reagent (10% v/v) was added to each well and the plate incubated for 2 h. Fluorescence emission was measured using a microplate reader (Tecan; ex: 540 nm; em: 590 nm).

### Example 2: Synthesis of p-Nitrobenzyloxycarbonyl Coumarin

7-amino-4-methylcoumarin (200 mg, 1.14 mmol) was dissolved in 5 mL of dry DCM under N₂ atmosphere. Pyridine (100 µL, 1.24 mmol) was added and the solution was cooled to 0 °C. Subsequently, 4-Nitrobenzyl chloroformate (365 mg, 1.7 mmol) was added and the reaction stirred overnight. 20 mL of 0.5 M HCl was added which lead to the precipitation of the product. The precipitate was filtered off and washed using Et₂O. After drying, 212 mg of product was obtained (53 % yield).

### Example 3: Synthesis of p-Nitrobenzyloxycarbonyl Gemcitabine

Gemcitabine hydrochloride (299 mg, 1 mmol) was dissolved in dry DMA (4 mL) with NaHCO₃ (252 mg, 3 mmol) under N₂ atmosphere. The mixture was then cooled to 0 °C in an ice bath. 4-Nitrobenzyl chloroformate (324 mg, 1.5 mmol) was subsequently added to the mixture. The mixture was stirred overnight at room temperature. The solution was filtered and the filtrate was concentrated *in vacuo.* The filtrate was added in its liquid form to a silica column (66 g). The column was run with 500 mL of a mixture of hexane/ethyl acetate (20:80), followed by 500 mL of ethyl acetate.

### Example 4: Synthesis of 1-(5-nitrothiophen-2-yl)ethyl Methotrexate

1-(5-nitrothien-2-yl)ethan-1-ol (130 mg, 0.75 mmol), methotrexate (227.2 mg, 0.5 mmol), pyridine (125 µL, 1.5 mmol) were dissolved in dry DCM (2 mL) under N₂ atmosphere. The mixture was then cooled to 0 °C in an ice bath. A solution of phosgene (428 µL, 0.6 mmol, 15 wt. % in toluene) was added dropwise to the reaction mixture. The mixture was stirred overnight at room temperature. The reaction mixture was partitioned with ethyl acetate and water. The aqueous phase was extracted with ethyl acetate and the organic phases were combined and then washed with brine the dried with MgSO₄. The solution was filtered and the filtrate was concentrated *in vacuo.* The filtrate was added in its liquid form to a silica column (30 g). The column was run with 250 mL of a mixture of hexane/ethyl acetate (20:80), followed by 250 mL of ethyl acetate.

### Example 5: Synthesis of 2-methoxy-4-nitrobenzyl Methotrexate

(2-methoxy-4-nitrophenyl)methanol (137.4 mg, 0.75 mmol), methotrexate (227.2 mg, 0.5 mmol), pyridine (125 µL, 1.5 mmol) were dissolved in dry DCM (2 mL) under N₂ atmosphere. The mixture was then cooled to 0 °C in an ice bath. A solution of phosgene (428 µL, 0.6 mmol, 15 wt. % in toluene) was added dropwise to the reaction mixture. The mixture was stirred overnight at room temperature. The reaction mixture was partitioned with ethyl acetate and water. The aqueous phase was extracted with ethyl acetate and the organic phases were combined and then washed with brine the dried with MgSO₄. The solution was filtered and the filtrate was concentrated *in vacuo.* The filtrate was added in its liquid form to a silica column (30 g). The column was run with 250 mL of a mixture of hexane/ethyl acetate (20:80), followed by 250 mL of ethyl acetate.

### Example 6: Kinetics of Deprotection (coumarin)

The kinetics of the reaction was first investigated and a model of the prodrug system was developed with 7-amino-4-methylcoumarin. Such a model allows for facile and rapid kinetics study since 7-amino-4-methylcoumarin is a fluorophore in its deprotected form while in its protected form its fluorescence is quenched. The reaction kinetics was measured in two important media for cellular culture. Namely, the Minimum Essential Medium Eagle (or MEM) and Ham's F-12K (Kaighn's) Medium (or F-12K) respectively. The *t*_{1/2} is approximately 10 minutes for both medium. Given the fact that the reaction is not stirred mechanically, that the hydrogen uptake is diffusion-controlled and that the reaction is done in a complex environment containing a plethora of competing substrates, such an expeditious kinetics makes this reaction propitious for our application.

Beyond the spatial control provided through local administration of the nanoparticles, another important characteristic of our system is its temporal control. In order to test this, the model reaction was used to run an "interval kinetics" experiment. Much like during interval trainings, the microplates were experiencing two alternating regimens: one with a hydrogen atmosphere and one with the usual incubator atmosphere. At the end of each interval, the fluorescence was measured. Unsurprisingly, after incubation in the usual incubator atmosphere, the reaction was virtually stopped. The little activity observed is likely to be due to dissolved hydrogen gas remaining in the medium after leaving the gas chamber open for equilibration.

### Example 7: Kinetics of Deprotection (gemcitabine)

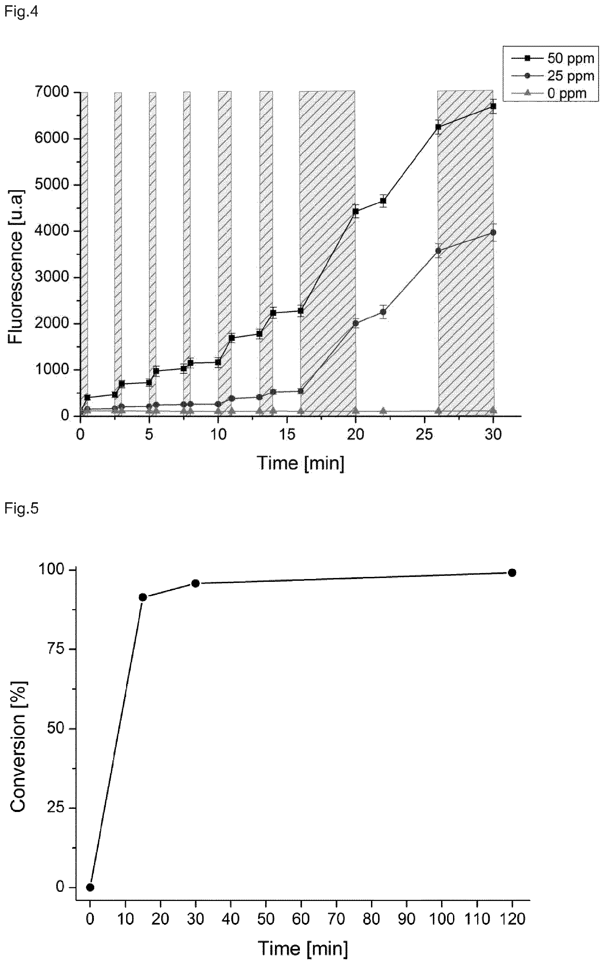

The actual drug/prodrug system being chemically slightly different from the model, the reaction kinetics experiments were repeated for the system before being tested on cells. The gemcitabine prodrug was deprotected in slightly different conditions than the model (lower substrate and catalyst concentrations). The conversion was measured by HPLC-MS where the amount of prodrug left in the supernatant was assessed. As can be seen on Fig. 5, the half-life remains similar to the one exhibited by the reaction model.

### Example 8: EC₅₀ Experiments

One of the foundation of the drug/prodrug system is that the prodrug is less effective than its deprotected counterpart. Dose-response experiments were performed in order to determine the half maximal effective concentration (EC₅₀) of both the drug as well as the prodrug. It was found that their values differ by about 5 orders of magnitude.

### Example 9: Extracellular Prodrug Activation

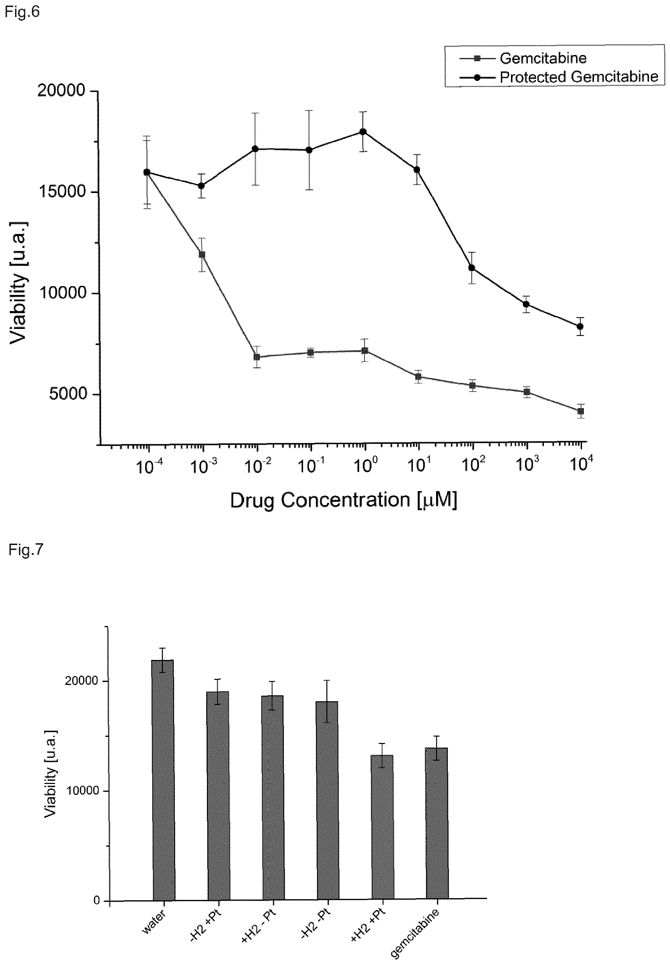

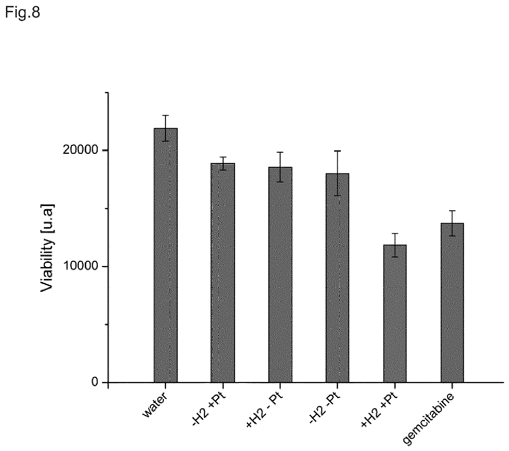

In order to prove that the activity is fully restored after the deprotection, an experiment was designed where the gemcitabine prodrug was treated with the four conditions of the square matrix defined by the two variables (presence of hydrogen and presence of a catalyst). As can be observed on Fig. 7 and Fig. 8, the activity of the drug is only recovered if both the hydrogen and the platinum nanoparticles are present. A small decrease is observed for the 3 conditions where either hydrogen and/or platinum is missing. This could be due to a constitutively occurring slow reduction of the nitro group by the cells. On both graphs, it can be seen that the conditions with both hydrogen and platinum have a slightly lower viability than the positive control with gemcitabine. Although this would require further investigations, it could be a consequence of the increased lipophilicity of the protected drug thereby conferring an increased uptake of the drug with respect to its deprotected counterpart.

## Claims

1. A prodrug compound consisting of
a. a drug moiety comprising an amine or hydroxyl moiety,
b. a protecting group moiety covalently linked to said amine or hydroxyl moiety,
wherein the protecting group moiety is cleavable from said amine or hydroxyl moiety by reaction with molecular hydrogen in the presence of a catalyst,
wherein said drug moiety is effective in the treatment of cancer when the drug moiety is separated from the protecting group
or a pharmaceutically acceptable salt of said prodrug compound.

2. The prodrug compound or its pharmaceutically acceptable salt of claim 1, wherein said drug moiety is known to interact with a therapeutic target, and said drug moiety comprises an interaction site that is the site where said drug moiety interacts with said therapeutic target, and said amine or hydroxyl moiety is located in or near said interaction site.

3. The prodrug compound or its pharmaceutically acceptable salt of claim 1 or 2,
wherein said protecting group moiety is selected from the group consisting of 4-nitrobenzylcarbamate, 1-(4-nitrophenyl)ethylcarbamate, 2-methoxy-4-nitrobenzylcarbamate, 2-(2-methoxyethoxy)-4-nitrobenzylcarbamate, 2-(2,3-dihydroxypropoxy)-4-nitrobenzylcarbamate, 2-(3-hydroxypropoxy)-4-nitrobenzylcarbamte, (5-nitrothiophen-2-yl)methylcarbamate, 1-(5-nitrothiophen-2-yl)ethylcarbamate, (1-methyl-2-nitro-1H-imidazol-5-yl)methylcarbamate, (1-methyl-5-nitro-1H-imidazol-2-yl)methylcarbamate, (5-nitrofuran-2-yl)methylcarbamate, (5-methoxy-1,3-dimethyl-4,7-dioxo-4,7-dihydro-1H-indol-2-yl)methylcarbamate and ethyl 4-((carbamoyloxy)methyl)-1-methyl-5-nitro-1 H-pyrrole-3-carboxylate.

4. The prodrug compound or its pharmaceutically acceptable salt according to any one of the preceding claims, wherein said catalyst is a platinum group metal selected from ruthenium, rhodium, palladium, osmium, iridium and platinum, in particular said catalyst is selected from platinum and palladium.

5. The prodrug compound or its pharmaceutically acceptable salt according to any one of the preceding claims, wherein said drug moiety is selected from the list comprising afatinib dimaleate, alectinib, brigatinib, ceritinib, crizotinib, docetaxel, doxorubicin hydrochloride, erlotinib hydrochloride, etoposide, etoposide phosphate, everolimus, gefitinib, gemcitabine hydrochloride, methotrexat, osimertinib, paclitaxel, pemetrexel disodium, topotecan hydrochloride, irinotecan and vinorelbine tartrate.

6. The prodrug compound or its pharmaceutically acceptable salt according to any one of claims 1 to 5, wherein the prodrug compound is **characterized by** a formula (1) 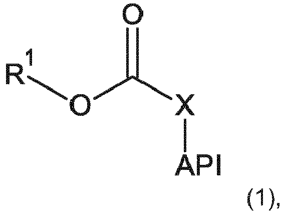 wherein API represents the drug moiety, X represents O or N (wherein API comprises a secondary amine represented by X and two bonds connect X with the API scaffold) or NH or NR² with R² being selected from C₁ to C₆ alkyl, and R¹-OCO represents the protecting group moiety.

7. The prodrug compound or its pharmaceutically acceptable salt according to claim 6, wherein R¹ is selected from a moiety **characterized by** any one of formulae (2a), (2b), (3), (4), (5), (6), (7), (8), (9), (10), (11), (12), (13), (14) or (15) 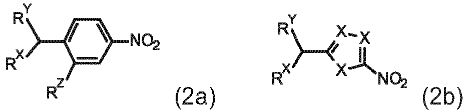 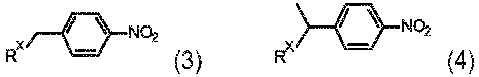 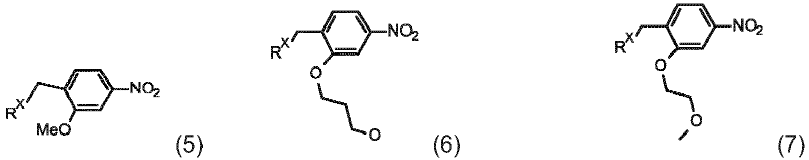 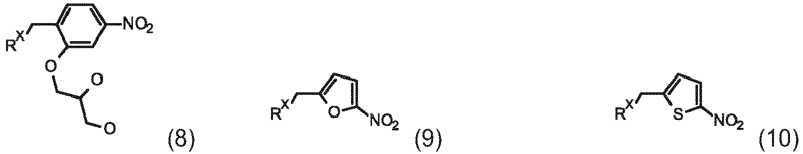 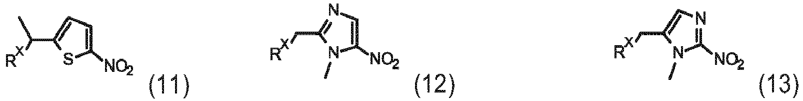 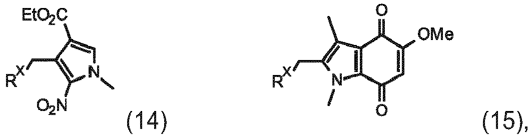 wherein
R^{Y} is a substituted or unsubstituted alkyl, particularly a substituted or unsubstituted C₁ to C₁₂ alkyl, more particularly a substituted or unsubstituted C₁ to C₆ alkyl,
R^{Z} is selected from C₁ to C₆ alkyl,
X is selected from O, S, NH or NR³ with R³ being selected from C₁ to C₆ alkyl and
R^{X} represents OCO-X-API.

8. The prodrug compound or its pharmaceutically acceptable salt according to claim 6 or 7, wherein API is selected from the list comprising a compound **characterized by** any one of formulae (16), (17), (18), (19), (20), (21), (22), (23), (24), (25), (26), (27), (28), (29), (30), (31), (32) or (33).

9. A prodrug compound or its pharmaceutically acceptable salt **characterized by** any one of formulae (16a), (17a), (18a), (18b) (19a), (20a), (21a), (22a), (23a), (24a), (24b), (24c), (25a), (26a), (27a), (28a), (28b), (29a), (30a), (31a), (32a), (32b) or (33a).

10. A combination medicament comprising
a. the prodrug compound or its pharmaceutically acceptable salt according to any one of claims 1 to 9,
b. a catalyst capable of effecting said reductive deprotection of said drug moiety, and optionally
c. a reducing agent, particularly hydrogen H₂.

11. The combination medicament according to claim 10, wherein
a. said prodrug compound or its pharmaceutically acceptable salt is formulated for systemic delivery, and
b. said catalyst is formulated for locally restricted delivery, in particular for inhalation.

12. The combination medicament according to claim 10, wherein said prodrug compound or its pharmaceutically acceptable salt, and said catalyst are formulated for inhalation.

13. The combination medicament according to any one of claims 10 to 12, wherein said catalyst is a platinum group metal selected from ruthenium, rhodium, palladium, osmium, iridium and platinum, in particular from platinum and palladium.

14. The prodrug according to any one of claims 1 to 9, or a combination medicament according to any one of claims 10 to 13, for use in a method of treatment or prevention of cancer.

15. The prodrug or combination medicament according to claim 14, wherein said method of treatment or prevention comprises the steps of
a. inhalation of a catalyst effecting said reductive deprotection of said API;
b. administration of said prodrug; and optionally
c. inhalation of a reducing agent, in particular hydrogen.
